# EUROPÄISCHE PATENTSCHRIFT

(11) **EP 3 028 744 B1**
(45) Veröffentlichungstag und Bekanntmachung des Hinweises auf die Patenterteilung: **26.12.2018**
(21) Anmeldenummer: 15192235.8
(22) Anmeldetag: 30.10.2015
(51) Int. Cl.: A61Q 15/00, A61K 8/26, A61K 8/86, A61K 8/894, A61K 8/04, A61K 8/06

(54) **KOSMETISCHES AEROSOLSPRAY MIT FRISCHEEFFEKT**
COSMETIC AEROSOL SPRAY WITH FRESH EFFECT
SPRAY AEROSOL COSMETIQUE AYANT UN EFFET DE FRAICHEUR

(30) Priorität: 02.12.2014 DE 102014224680
(43) Veröffentlichungstag der Anmeldung: 08.06.2016
(73) Patentinhaber: Henkel AG & Co. KGaA, 40589 Düsseldorf (DE)
(72) Erfinder: DÖRING, Thomas, 41540 Dormagen (DE); SOLICH, Daniel, 40764 Langenfeld (DE)

(56) Entgegenhaltungen:
- EP-A2- 1 938 783
- WO-A1-2012/084972
- DE-A1-102010 040 121
- GB-A- 1 300 399
- US-A- 3 863 005
- US-A1- 2013 280 175

## Beschreibung

Die vorliegende Anmeldung beschreibt als Aerosol versprühbare kosmetische Wasser-in-ÖI-Emulsionen, die einen angenehmen Frischeeffekt mit trocken kühlender Wirkung aufweisen.
Ein Aerosol ist ein disperses System, bei dem ein Feststoff oder eine Flüssigkeit in einem Gas sehr fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel-Zubereitungsgemisch durch eine feine Düse, das Treibmittel verdunstet und hinterlässt das fein verteilte Sprühgut als Aerosol.
Ein technisch wichtiges Gebiet für die Anwendung kosmetischer Aerosolsprays ist der Bereich der deodorierenden kosmetischen Erzeugnisse. Gängige Deodorantsprayzusammensetzungen liegen dabei häufig als wasserfreie ethanolische Lösungen des deodorierenden Wirkstoffes vor. Ein Nachteil dieser ethanolischen Lösungen ist bei entsprechend prädisponierten Personen mit empfindlicher Haut eine hautreizende Wirkung. Auch auf durch eine Rasur mechanisch gereizter Haut führt die Applikation der gängigen ethanolischen Lösungen zu einem sehr unangenehmen Brennen. Ein weiterer Nachteil ethanolischer Lösungen ist, dass die Einarbeitung wasserhaltiger oder wasserlöslicher Wirkstoffe, insbesondere von Antitranspirant- bzw. Deodorant-Wirkstoffen oder haarwuchshemmenden Wirkstoffen, die nicht auch in Ethanol löslich sind, nicht oder nur sehr eingeschränkt möglich ist.
Zudem sind wäßrig-alkoholische Lösungen von Antitranspirantien nur in speziellen Behältern lager- und versprühbar, da es bei den herkömmlichen Systemen aus Aluminium oder Weißblech zu signifikanter Korrosion kommt.
Als alternative Angebotsform zu den ethanolischen Lösungen werden im Stand der Technik wasserfreie Suspensionen des pulverförmigen schweißreduzierenden Wirkstoffes, meist eines Aluminiumsalzes, neben dem Treibgas in einem flüssigen Träger, meist einem relativ flüchtigen Öl wie Cyclomethicone, diskutiert. Vor dem Versprühen muss die Suspension aufgeschüttelt werden.
Ein Nachteil dieser Suspensionsaerosole ist das Risiko, dass die Ventil- bzw. Düsenbohrungen bei höheren Einsatzkonzentrationen des Salzes verstopfen. Es gab daher Versuche, das Antitranspirant-Salz in gelöster Form zu versprühen. Jedoch verursacht die Konfektionierung wässriger Antitranspirant-Salzlösungen in Treibmittel-haltigen Metalldosen große Korrosionsprobleme bei der Aerosolverpackung, so dass selbst bei lackierten Spraydosen unweigerlich Korrosionserscheinungen an der Dose oder am Ventilteller auftreten.
Als weitere alternative Angebotsform beschreibt der Stand der Technik Wasser-in-ÖI-Emulsionen (W/O-Emulsionen). Schweißhemmende W/O-Emulsionssprays weisen besonders hautpflegende Eigenschaften auf. Beim Aufsprühen auf die Haut wird durch das Treibgas vorübergehend ein leicht kühlender Effekt erzeugt, der allerdings nicht anhält. Allerdings ist der kühlende Effekt, der durch die enthaltene wässrige Phase der Emulsion entsteht, bei einer W/O-Emulsion, bei der die wässrige Phase die innere Phase darstellt, kaum wahrnehmbar.
Es wäre daher wünschenswert, die Vorteile von Emulsionssprays mit dem angenehmen Hautgefühl alkoholischer Sprays verbinden zu können, ohne auf teure Spezialverpackungen zurückgreifen zu müssen.

Die DE 10 2010 040121 A1 offenbart in Aerosol-Abgabevorrichtungen verpackte Öl-in-Wasser-Emulsionen, die Treibmittel und weniger als 4 Gew.-% Ethanol enthalten.

In der WO2012/084972 A1 wird eine W/O-Emulsion mit Treibgas offenbart, welche Propandiol und Phenoxyethanol, aber kein Ethanol enthält.

Die US 3,863,005 A offenbart Alumniumchlorohydroxid-haltige Aerosolformulierungen, welche 14,5 Gew.-% Ethanol enthalten.

In der EP 1 938 783 A2 werden W/O-Emulsionen offenbart, die mindestens 10 Gew.-% Ethanol enthalten und in Form eines Aerosolsprays vorliegen.

Ein kosmetisches Produkt, umfassend eine W/O-Emulsion, welche Propandiol und Phenoxyethanol, aber kein Ethanol enthält, wird in der US2013/280175 A1 offenbart.

Die GB 1,300,399 A beschreibt hochethanolhaltige Aerosolzusammensetzungen.

Der vorliegenden Anmeldung lag die Aufgabe zugrunde, eine für die Anwendung in einem Aerosolspray geeignete stabile Wasser-in-Öl-Emulsion mit einem Gehalt an schweißhemmenden Aluminiumsalzen und Ethanol bereitzustellen, die einen angenehmen Kühl- und Frischeeffekt auf der Haut bewirkt und die vorstehend geschilderten Nachteile vermeidet.
Es wurde nun festgestellt, daß die aufgeführte Aufgabenstellung durch schweißhemmende Wasser-in-Öl-Emülsions-Sprays gelöst wird, die bestimmte Mengen an schweißhemmenden Aluminiumsalz und Ethanol und bestimmte Diole nur bis zu einer Maximalmenge enthalten.
Ein erster Gegenstand der vorliegenden Anmeldung ist daher ein kosmetisches Produkt, bestehend aus
- einer Wasser-in-ÖI-Emulsion, enthaltend - bezogen auf das Gewicht der Emulsion -
   a) 5 bis 35 Gew.-% mindestens eines schweißhemmenden Aluminiumsalzes,
   b) 5 bis 14,25 Gew.-% Ethanol,
   c) 0 bis 13 Gew.-% 1,2-Propandiol,
- mindestens einem Treibmittel,
- einer Aerosol-Abgabevorrichtung.
Sofern nicht anders angegeben, beziehen sich alle Mengenangaben in dieser Anmeldung auf das Gewicht der erfindungsgemäßen Treibmittel-freien Wasser-in-ÖI-Emulsion.
Die erfindungsgemäßen Wasser-in-Öl-Emulsionen enthalten mindestens ein schweißhemmendes Aluminiumsalz.
Bevorzugte schweißhemmende Aluminiumsalze sind ausgewählt aus den wasserlöslichen adstringierenden anorganischen und organischen Salzen des Aluminiums bzw. beliebigen Mischungen dieser Salze. Alumosilicate und Zeolithe zählen erfindungsgemäß nicht zu den schweißhemmenden Aluminiumsalzen. Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.
Besonders bevorzugte schweißhemmende Aluminiumsalze sind ausgewählt aus Aluminiumchlorhydrat, insbesondere Aluminiumchlorhydrat mit der allgemeinen Formel [Al₂(OH)₅Ct · 1-6 H₂O]ₙ, bevorzugt [Al₂(OH)₅Cl · 2-3 H₂O]ₙ, das in nicht-aktivierter oder in aktivierter (= depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (= depolymerisierter) Form vorliegen kann.
Weiterhin bevorzugt sind Aluminiumsesquichlorhydrat, Aluminiumdichlorhydrat, oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG, Aluminiumsesquichlorohydrex-PEG oder Aluminium-PEG-dichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat.
Erfindungsgemäß besonders bevorzugte schweißhemmende Aluminiumsalze sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.
Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.
Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.
Für die Stabilisierung der schweißhemmenden Salze bevorzugte Hydroxyalkansäuren sind ausgewählt aus Glycolsäure und Milchsäure.
Weitere bevorzugte schweißhemmende Wirkstoffe sind aktivierte Aluminiumsalze, enthaltend, bezogen auf ihr Gewicht, 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.
Besonders bevorzugte feste schweißhemmende aktivierte Salzzusammensetzungen enthalten, bezogen auf ihr Gewicht, 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 -1:10, beträgt.
Weitere besonders bevorzugte feste schweißhemmende aktivierte Salzzusammensetzungen enthalten, bezogen auf ihr Gewicht, 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 -1:20, bevorzugt 1:1 -1:10, beträgt.

Weitere besonders bevorzugte feste schweißhemmende aktivierte Salzzusammensetzungen enthalten, bezogen auf ihr Gewicht, 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.
Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von AI:X 0,9:1 bis 2,1:1 beträgt. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.
Besonders bevorzugte erfindungsgemäße Produkte sind dadurch gekennzeichnet, daß die Emulsion 6 bis 32,5 Gew.-%, bevorzugt 7 bis 30 Gew.-%, besonders bevorzugt 8 bis 27,5 Gew.-%, außerordentlich bevorzugt 9 bis 26 Gew.-% und insbesondere 10 bis 25 Gew.-%, jeweils bezogen auf das Gesamtgewicht der kristallwasser- und ligandenfreien Aktivsubstanz (USP) in der treibmittelfreien Wasser-in-ÖI-Emulsion, schweißhemmende(s) Aluminiumsalz(e) enthält.

In einer besonders bevorzugten Ausführungsform enthält die Wasser-in-ÖI-Emulsion ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. In einer weiteren besonders bevorzugten Ausführungsform enthält die Wasser-in-ÖI-Emulsion ein nicht-aktiviertes adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nichtaktivierte Aluminiumchlorohydrate sind beispielsweise Chlorhydrol® (Summit-Reheis), ACH 303 (Summit-Reheis), Locron® L (Clariant) oder Aloxicoll® L (Giulini). Eine bevorzugte aktivierte Aluminiumchlorohydrat-Lösung ist z. B. Reach® 501 von Summit-Reheis.
Besonders bevorzugte erfindungsgemäße Produkte sind dadurch gekennzeichnet, daß die Emulsion 6 bis 32,5 Gew.-%, bevorzugt 7 bis 30 Gew.-%, besonders bevorzugt 8 bis 27,5 Gew.-%, außerordentlich bevorzugt 9 bis 26 Gew.-% und insbesondere 10 bis 25 Gew.-%, jeweils bezogen auf das Gesamtgewicht der kristallwasser- und ligandenfreien Aktivsubstanz (USP) in der treibmittelfreien Wasser-in-ÖI-Emulsion, Aluminiumchlorohydrat enthält.

Ein bevorzugter Gegenstand der vorliegenden Anmeldung ist ein kosmetisches Produkt, bestehend aus
- einer Wasser-in-ÖI-Emulsion, enthaltend
   a) 6 bis 32,5 Gew.-%, bevorzugt 7 bis 30 Gew.-%, besonders bevorzugt 8 bis 27,5 Gew.-%, außerordentlich bevorzugt 9 bis 26 Gew.-% und insbesondere 10 bis 25 Gew.-%, jeweils bezogen auf das Gesamtgewicht der kristallwasser- und ligandenfreien Aktivsubstanz (USP) in der treibmittelfreien Wasser-in-Öl-Emulsion, Aluminiumchlorohydrat,
   b) 5 bis 14,25 Gew.-% Ethanol,
   c) 0 bis 13 Gew.-% 1,2-Propandiol,
- mindestens einem Treibmittel,
- einer Aerosol Abgabevorrichtung.

Die erfindungsgemäßen Wasser-in-ÖI-Emulsionen enthalten als zweiten wesentlichen Bestandteil Ethanol. Dies läßt sich in der erfindungsgemäßen Formulierung so einarbeiten, daß die Emulsion stabil bleibt und sowohl Ethanol als auch Aluminiumsalz in der inneren Wasserphase lokalisiert sind, so daß ein direkter Kontakt beider Komponenten mit dem Behälter- oder Ventilmaterial ausgeschlossen ist. Korrosionsprobleme sind dabei auch bei Verwendung der üblichen Ventil- oder Behältermaterialien wie Aluminium oder Weißblech nicht zu befürchten.

Besonders bevorzugte erfindungsgemäße Produkte sind dadurch gekennzeichnet, daß die Emulsion 6 bis 14 Gew.-%, außerordentlich bevorzugt 7,5 bis 13,5 Gew.-% und insbesondere 10 bis 13 Gew.-% Ethanol enthält.

Die im erfindungsgemäßen Produkt enthaltene Emulsion beinhaltet maximal 13 Gew.-% 1,2-Propandiol. Besonders bevorzugt sind einerseits völlig propandiolfreie Emulsionen, andererseits solche, welche 1,2-Propandiol in engeren Mengenbereichen enthalten.

Sofern aus formulierungstechnischen Gründen ein Gehalt an 1,2-Propandiol gewünscht ist, sind erfindungsgemäße Produkte bevorzugt, bei denen die Emulsion 0,5 bis 12 Gew.-%, bevorzugt 1 bis 11 Gew.-%, weiter bevorzugt 2 bis 10 Gew.-%, besonders bevorzugt 3 bis 9 Gew.-%, außerordentlich bevorzugt 4 bis 8 Gew.-% und insbesondere 5 bis 7 Gew.-% 1,2-Propandiol enthält.

Sofern auf 1,2-Propandiol verzichtet werden kann, sind erfindungsgemäße Produkte bevorzugt, bei denen die Emulsion weniger als 1 Gew.-%, bevorzugt weniger als 0,75 Gew.-%, weiter bevorzugt weniger als 0,5 Gew.-%, besonders bevorzugt weniger als 0,25 Gew.-%, außerordentlich bevorzugt weniger als 0,1 Gew.-% und insbesondere weniger als 0,01 Gew.-% 1,2-Propandiol enthält.

Die im erfindungsgemäßen Produkt enthaltene Emulsion kann mindestens einen Wasser-in-ÖI-Emulgator enthalten. Als Wasser-in-ÖI-Emulgator wird im Sinne der vorliegenden Anmeldung eine grenzflächenaktive Substanz angesehen, die unter Normalbedingungen einen HLB-Wert im Bereich von 1,5 bis 6,5, bevorzugt 4 bis 6,5, aufweist. "Normalbedingungen" sind im Sinne der vorliegenden Anmeldung eine Temperatur von 20 °C und ein Druck von 1013,25 hPa. Schmelzpunktangaben beziehen sich ebenfalls auf einen Druck von 1013,25 hPa. Wasser-in-ÖI-Emulsionen, bei denen der mindestens eine Wasser-in-ÖI-Emulgator b) in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-ÖI-Emulsion, enthalten ist, sind erfindungsgemäß bevorzugt.
Erfindungsgemäß bevorzugte Wasser-in-ÖI-Emulgatoren sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (frühere INCI-Bezeichnung Cetyl Dimethicone Copolyol), zum Beispiel erhältlich als Abil EM 90 oder in einer Mischung aus Polyglyceryl-4-isostearat, Cetyl PEG/PPG-10/1 Dimethicone und Hexyllaurat unter dem Handelsnamen Abil WE 09 von der Firma Evonik, sowie weiterhin bevorzugt Lauryl PEG/PPG-18/18 Methicone, zum Beispiel erhältlich als Dow Corning 5200 Formulation Aid.
Eine erfindungsgemäß besonders bevorzugte Gruppe von Wasser-in-ÖI-Emulgatoren sind die Poly-(C₂-C₃)alkylenglycol-modifizierten Silicone, deren frühere INCI-Bezeichnung Dimethicone Copolyol lautete, bevorzugt Poly-(C₂-C₃)alkylenglycol-modifizierte Silicone mit den INCI-Bezeichnungen PEG-x Dimethicone mit x = 2 - 20, bevorzugt 3 - 17, besonders bevorzugt 11 - 12, Bis-PEG-y Dimethicone mit y = 3 - 25, bevorzugt 4 - 20, PEG/PPG a/b Dimethicone, wobei a und b unabhängig voneinander für Zahlen von 2 - 30, bevorzugt 3 - 24, besonders bevorzugt 12 - 20, insbesondere 14 - 18, stehen, Bis-PEG/PPG-c/d Dimethicone, wobei c und d unabhängig voneinander für Zahlen von 10 - 25, bevorzugt 14 - 20 und besonders bevorzugt 14 - 16, stehen, und Bis-PEG/PPG-e/f PEG/PPG g/h Dimethicone, wobei e, f, g und h unabhängig voneinander für Zahlen von 10 - 20, bevorzugt 14 - 18 und besonders bevorzugt 16, stehen, mit der Bedingung, dass diese ethoxylierten und/oder propoxylierten Siliconemulgatoren unter Normalbedingungen einen HLB-Wert im Bereich von 1,5 bis 6,5, bevorzugt 4 bis 6,5, aufweisen.
Erfindungsgemäß bevorzugte Produkte sind dadurch gekennzeichnet, daß die Wasser-in-ÖI-Emulsion mindestens einen Wasser-in-ÖI-Emulgator, ausgewählt aus der Gruppe von Poly-(C₂-C₃)alkylenglycol-modifizierten Siliconen mit den INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12-20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen) in einer Menge von 0,5 - 5 Gew.-%, bezogen auf das Gesamtgewicht der Emulsion, enthält.

Zur Klarstellung sei angemerkt, dass diese vorgenannten bevorzugten Wasser-in-ÖI-Emulgatoren nur mit Methylgruppen alkyliert sind und - wie die Bezeichnungen "Dimethicone Copolyol" und "PEG/PPG Dimethicone" bereits implizieren - keine höheren Alkylgruppen, z. B. Ethyl, Lauryl, Cetyl, im Molekül aufweisen.
Ein erfindungsgemäß außerordentlich bevorzugter Wasser-in-ÖI-Emulgator ist PEG/PPG-18/18 Dimethicone, das einen HLB-Wert im Bereich von 4 bis 6,5 aufweist. Dieser besonders bevorzugte Wasser-in-ÖI-Emulgator PEG/PPG-18/18 Dimethicone ist im Handel üblicherweise nicht als Reinsubstanz, sondern in einem kosmetischen Öl dispergiert erhältlich. Bevorzugte erfindungsgemäße Produkte umfassen eine Wasser-in-ÖI-Emulsion, enthaltend den Wasser-in-ÖI-Emulgator PEG/PPG-18/18 Dimethicone als Dispersion in Dimethicone, das eine Viskosität von 3 - 100 cSt, bevorzugt 5 - 50 cSt, besonders bevorzugt 10 - 20 cSt (jeweils bei 25°C), aufweist. Ein erfindungsgemäß besonders bevorzugtes Handelsprodukt ist Dow Corning ES-5227 DM von der Firma Dow Corning. Alternativ kann der Emulgator auch in Cyclopentasiloxan dispergiert vorliegen. Ein bevorzugtes Handelsprodukt ist hier Dow Corning 5225 C von der Firma Dow Corning. Wasser-in-ÖI-Emulsionen, bei denen der Wasser-in-ÖI-Emulgator nur ausgewählt ist aus PEG/PPG-18/18 Dimethicone, das einen HLB-Wert im Bereich von 4 bis 6,5 aufweist und das in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-ÖI-Emulsion, enthalten ist, sind erfindungsgemäß besonders bevorzugt.
Die erfindungsgemäßen Wasser-in-ÖI-Emulsionen können darüber hinaus mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17 enthalten.
Bevorzugte erfindungsgemäße Wasser-in-ÖI-Emulsionen sind dadurch gekennzeichnet, dass der mindestens eine Öl-in-Wasser-Emulgator oder Lösungsvermittler einen HLB-Wert > 9, bevorzugt einen HLB-Wert > 12 bis 19 und insbesondere einen HLB-Wert > 14 bis 18 aufweist. Besonders bevorzugte Öl-in-Wasser-Emulgatoren oder Lösungsvermittler weisen einen HLB-Wert von 15 bis 17 auf.
Die Ermittlung des HLB-Wertes erfolgt erfindungsgemäß nach Griffin gemäß der Formel: HLB = 20 * (Mₕ / M), wobei Mₕ die Molmasse des hydrophilen Anteils eines Moleküls ist und M die Molmasse des gesamten Moleküls. Es ergibt sich damit eine Skala von 0 bis 20.
Bevorzugte erfindungsgemäße Wasser-in-ÖI-Emulsionen sind dadurch gekennzeichnet, dass der mindestens eine Öl-in-Wasser-Emulgator oder Lösungsvermittler ausgewählt ist aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und verestert mit 1 bis 4 gesättigten oder ungesättigten, linearen oder verzweigten, gegebenenfalls hydroxylierten C₈ - C₃₀-Fettsäureresten, sofern diese Polyglycerinpartialester einen HLB-Wert im Bereich von > 7 bis 20 aufweisen, sowie Mischungen der vorgenannten Substanzen.
Die ethoxylierten C₈-C₂₄-Alkanole haben die Formel R¹O(CH₂CH₂O)ₙH, wobei R¹ für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid steht.
Erfindungsgemäß besonders bevorzugt sind Adduktverbindungen von 10 - 100, bevorzugt 10 - 30 Mol Ethylenoxid, an 1 Mol Alkanol, ausgewählt aus Caprylalkohol, 2-Ethylhexylalkohol, Caprinalkohol, Laurylalkohol, Isotridecylalkohol, Myristylalkohol, Cetylalkohol, Palmitoleylalkohol, Isocetylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid, bevorzugt 10 - 30 Mol Ethylenoxid, an 1 Mol Alkanol, ausgewählt aus technischen Fettalkoholen mit 12 - 18 Kohlenstoffatomen, wie beispielsweise Kokos-, Palm-, Palmkern- oder Talgfettalkohol, sind erfindungsgemäß bevorzugt. Außerordentlich bevorzugt ist Isoceteth-20. Die vorgenannten ethoxylierten Alkanole sind nichtionisch und werden auch als Polyethylenglycolether oder nichtionische Polyethylenglycolether bezeichnet.
Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 50 Mol Ethylenoxid, an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100, vorzugsweise 10 - 50, Mol Ethylenoxid an 1 Mol Säure, ausgewählt aus technischen Fettsäuren mit 12 - 18 Kohlenstoffatomen, wie Kokos-, Palm-, Palmkern- oder Talgfettsäure, sind bevorzugt. Besonders bevorzugt sind PEG-50-monostearat, PEG-100-monostearat, PEG-50-monooleat, PEG-100-monooleat, PEG-50-monolaurat und PEG-100-monolaurat.
Besonders bevorzugt sind die C₁₂-C₁₈-Alkanole mit 10 - 30 Einheiten Ethylenoxid pro Molekül sowie Mischungen dieser Substanzen. Außerordentlich bevorzugt sind die C₁₄-C₁₈-Alkanole mit 200 - 30 Einheiten Ethylenoxid pro Molekül sowie Mischungen dieser Substanzen. Außerordentlich bevorzugt sind insbesondere Isoceteth-12, Isoceteth-20, Isoceteth-30, Isosteareth-12, Isosteareth-20, Isosteareth-30, Laureth-12 und Beheneth-20.
Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, bevorzugt 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.
Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Als Öl-in-Wasser-Emulgatoren geeignete ethoxylierte Sterine sind z. B. PEG-10 Soy Sterol, PEG-16 Soy Sterol und PEG-25 Soy Sterol. Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert im Bereich von > 7 bis 20 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.
Bevorzugt ist der mindestens eine Öl-in-Wasser-Emulgator oder Lösungsvermittler mit einem HLB-Wert im Bereich von > 7 bis 20 ausgewählt aus nichtionischen Polyethylenglycolethern mit einem entsprechenden HLB-Wert.
Erfindungsgemäß besonders bevorzugte Produkte enthalten mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf das Gewicht der gesamten treibmittelfreien Wasser-in-ÖI-Emulsion. Weitere besonders bevorzugte Produkte enthalten mindestens einen nichtionischen Polyethylenglycolether mit einem HLB-Wert im Bereich von > 7 - 20, bevorzugt im Bereich von > 12 - 19, besonders bevorzugt im Bereich von > 14 - 18 und außerordentlich bevorzugt im Bereich von 15 bis 17, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-ÖI-Emulsion. Weitere besonders bevorzugte Produkte enthalten Isoceteth-20 in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion.
Die erfindungsgemäßen Wasser-in-ÖI-Emulsionen können zusätzlich mindestens ein kosmetisches Öl enthalten, das kein Riechstoff und kein ätherisches Öl ist. Das kosmetische Öl ist unter Normalbedingungen flüssig. Unter ätherischen Ölen werden erfindungsgemäß Gemische aus flüchtigen Komponenten verstanden, die durch Wasserdampfdestillation aus pflanzlichen Rohstoffen hergestellt werden, wie beispielsweise Citrusöle.
Erfindungsgemäß bevorzugte Produkte enthalten mindestens ein kosmetisches Öl, das kein Riechstoff und kein ätherisches Öl ist, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-ÖI-Emulsion.
Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.
Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.
Cyclomethicone sind im Stand der Technik als gut geeignete Öle für kosmetische Produkte, insbesondere für schweißhemmende und deodorierende Produkte, wie Sprays und Stifte, bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen Produkte maximal 1 Gew.-%, bevorzugt maximal 0,1 Gew.-%, Cyclomethicone, jeweils bezogen auf das Gewicht der Wasser-in-ÖI-Emulsion, ohne das Gewicht des Treibmittels zu berücksichtigen.
Ein erfindungsgemäß bevorzugter Cyclomethicone-Ersatzstoff ist eine Mischung aus C₁₃-C₁₆-Isoparaffinen, C₁₂ - C₁₄-Isoparaffinen und C₁₃ - C₁₅-Alkanen, deren Viskosität bei 25°C im Bereich von 2 bis 6 mPas liegt und die einen Dampfdruck bei 20°C im Bereich von 10 bis 150 Pa, bevorzugt 100 bis 150 Pa, aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning® 200 (0,65 cSt) und Dow Corning® 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.
Bevorzugte erfindungsgemäße Antitranspirant-Zusammensetzungen enthalten wegen des trockeneren Hautgefühls und der schnelleren Wirkstofffreisetzung mindestens ein flüchtiges Siliconöl, das cyclisch oder linear sein kann.
Weitere bevorzugte erfindungsgemäße Produkte enthalten wegen des trockeneren Hautgefühls und der schnelleren Wirkstofffreisetzung mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa. Dieses mindestens eine C₈-C₁₆-Isoparaffin ist bevorzugt in einer Gesamtmenge von 1 - 30 Gew.-%, bevorzugt 3 - 26 Gew.-%, besonders bevorzugt 5 - 24 Gew.-%, außerordentlich bevorzugt 8 - 17 Gew.-%, enthalten, jeweils bezogen auf das Gewicht der Wasser-in-ÖI-Emulsion, ohne das Gewicht des Treibmittels zu berücksichtigen.
Weitere erfindungsgemäß bevorzugte Wasser-in-ÖI-Emulsionen enthalten mindestens ein nichtflüchtiges kosmetisches Öl, ausgewählt aus nichtflüchtigen Siliconölen und nichtflüchtigen Nichtsiliconölen. Rückstände des gelösten Antitranspirantwirkstoffes, aber auch Rückstände von in der Emulsion unlöslichen Bestandteilen, wie Talkum oder Silicapartikel, können erfolgreich mit einem nichtflüchtigen Öl maskiert werden. Außerdem können mit einem Gemisch aus verschiedenen Ölen, insbesondere aus nichtflüchtigem und flüchtigem Öl, Parameter wie Hautgefühl, Sichtbarkeit des Rückstands und Stabilität der erfindungsgemäßen Zusammensetzung feinreguliert und besser an die Bedürfnisse der Verbraucher angepasst werden.
Erfindungsgemäß bevorzugte Produkte sind dadurch gekennzeichnet, daß die Wasser-in-ÖI-Emulsion eine Ölphase von 1 - 60 Gew.-%, bezogen auf das Gesamtgewicht der Emulsion, enthält, wobei die Ölphase zu mindestens 90 Gew.-% aus bei 20 °C flüssigen Ölkomponenten besteht.

Erfindungsgemäß besonders bevorzugte Produkte sind dadurch gekennzeichnet, daß die Wasser-in-ÖI-Emulsion 1 - 60 Gew.-%, vorzugsweise 2,5 bis 55 Gew.-%, weiter bevorzugt 5 bis 50 Gew.-%, besonders bevorzugt 7,5 bis 45 Gew.-% und insbesondere 10 bis 40 Gew.-% mindestens eines Öls aus der Gruppe Decamethylcyclopentasiloxan, Dodecamethylcyclohexasiloxan, Hexamethyldisiloxan (L₂), Octamethyltrisiloxan (L₃), Decamethyltetrasiloxan (L₄), beliebigen Zweier- und Dreiermischungen aus L₂, L₃ und/ oder L₄, weiterhin 2-Ethylhexylpalmitat, Hexyldecyllaurat, 2-Ethylhexylstearat, 2-Ethylhexyllaurat, Isopropylmyristat, Isopropylpalmitat, C₁₂-C₁₅-Alkylbenzoat, C₁₂-C₁₅-Alkyllactat, Di-C₁₂-C₁₃-Alkylmalat, PPG-14-Butylether, Isododecan, Isohexadecan, Isoeicosan, Polyisobuten und Polydecene sowie Mischungen der genannten Komponenten, enthält. Selbstverständlich ist es ebenfalls möglich, erfindungsgemäße Produkte mit einem geringen Anteil an flüchtigen Ölen - das heißt, mit 0,5 - 15 Gew.-% an flüchtigen Ölen, bezogen auf das Gewicht der treibmittelfreien Wasser-in-ÖI-Emulsion - oder sogar ohne flüchtige Öle zu formulieren.
Als kosmetisches Öl d) erfindungsgemäß weiterhin besonders bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctan-säure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat, Ethylenglycoldipalmitat. n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv® TN (C₁₂-C₁₅-Alkylbenzoat), sowie Benzoesäureisostearylester, z. B. erhältlich als Finsolv® SB, 2-Ethylhexylbenzoat, z. B. erhältlich als Finsolv® EB, und Benzoesäure-2-octyldodecylester, z. B. erhältlich als Finsolv® BOD.
Als für die Emulsionsstabilität und Wirkstofffreisetzung besonders vorteilhaft hat sich der Einsatz von Isopropylestern von C₁₂-C₁₈-Carbonsäuren, insbesondere der Einsatz von Isopropylmyristat, und besonders bevorzugt Mischungen von Isopropylmyristat mit C₁₀-C₁₃-Isoparaffin-Mischungen, letztere bevorzugt mit einem Dampfdruck bei 20°C von 10 - 400 Pa, erwiesen.
Ein weiteres besonders bevorzugtes Esteröl ist Triethylcitrat. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine. Weitere erfindungsgemäß bevorzugte Produkte enthalten Triethylcitrat und eine Mischung aus Isodecan, Isoundecan, Isododecan und Isotridecan.
Erfindungsgemäß bevorzugte Wasser-in-ÖI-Emulsionen enthalten mindestens einen Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, in einer Gesamtmenge von 1 - 30 Gew.-%, bevorzugt 5 - 26 Gew.-%, besonders bevorzugt 9 - 24 Gew.-%, außerordentlich bevorzugt 12 - 17 Gew.-%, jeweils bezogen auf das Gewicht der gesamten treibmittelfreien Wasser-in-ÖI-Emulsion.
Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.
Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol® 318 oder Myritol® 331 (BASF/ Cognis) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in dem erfindungsgemäßen Produkt aus. Besonders bevorzugt beträgt das Gesamtgewicht an Triglyceridölen 0,5 - 10 Gew.-%, bevorzugt 1 - 5 Gew.-%, jeweils bezogen auf das Gewicht der gesamten Wasser-in-ÖI-Emulsion, ohne das Gewicht des Treibmittels zu berücksichtigen.
Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.
Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol® CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen.
Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf das Gewicht der gesamten Wasser-in-ÖI-Emulsion, ohne das Gewicht des Treibmittels zu berücksichtigen.
Weitere kosmetische Öle, die erfindungsgemäß besonders bevorzugt sind, sind ausgewählt aus nichtflüchtigen Siliconölen. Erfindungsgemäß bevorzugte nichtflüchtige Siliconöle sind ausgewählt aus linearen Polyalkylsiloxanen mit einer kinematischen Viskosität bei 25°C von mindestens 5 cSt bis 2000 cSt, insbesondere ausgewählt aus linearen Polydimethylsiloxanen mit einer kinematischen Viskosität bei 25°C von 5 cSt bis 2000 cSt, bevorzugt 10 bis 350 cSt, besonders bevorzugt 50 - 100 cSt, wie sie z. B. unter den Handelsnamen Dow Corning® 200 bzw. Xiameter PMX von Dow Corning bzw. Xiameter erhältlich sind. Weitere bevorzugte nichtflüchtige Siliconöle sind Phenyltrimethicone mit einer kinematischen Viskosität bei 25°C von 10 bis 100 cSt, bevorzugt von 15 - 30 cSt sowie Cetyldimethicone.
Erfindungsgemäß bevorzugte Wasser-in-ÖI-Emulsionen enthalten mindestens ein nichtflüchtiges Siliconöl, das bevorzugt ausgewählt ist aus linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 0,1 - 30 Gew.-%, bevorzugt 1 - 24 Gew.-%, besonders bevorzugt 2 - 18 Gew.-%, außerordentlich bevorzugt 4 - 10 Gew.-%, jeweils bezogen auf das Gewicht der gesamten treibmittelfreien Wasser-in-ÖI-Emulsion.
Die erfindungsgemäßen Produkte enthalten Wasser bevorzugt in einer Gesamtmenge von 5 - 55 Gew.-%, bevorzugt 10 - 40 Gew.-%, besonders bevorzugt 15 - 30 Gew.-%, außerordentlich bevorzugt 20 - 25 Gew.-%, jeweils bezogen auf das Gewicht der gesamten treibmittelfreien Wasser-in-Öl-Emulsion.
Die erfindungsgemäßen Produkte enthalten bevorzugt ein oder mehrere Konservierungsmittel. Erfindungsgemäß bevorzugte Konservierungsmittel sind Formaldehydabspalter (wie z. B. 1,3-Dimethylol-4,4-dimethylhydantoin, INCI-Bezeichnung DMDM Hydantoin), lodopropinylbutylcarbamate wie 3-lod-2-propinylbutylcarbamat, Parabene (d. h. para-Hydroxybenzoesäurealkylester, wie Methyl-, Ethyl-, Propyl- und/oder Butylparaben), Phenoxyethanol, Ethanol, Benzoesäure, Dibromdicyanobutan (2-Brom-2-brommethylglutarodinitril), 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate. Erfindungsgemäß besonders bevorzugte Konservierungsmittel sind ausgewählt aus Parabenen (Methyl-, Ethyl-, Propyl- und/oder Butylparaben) und/oder Phenoxyethanol.
Die Konservierungsmittel sind in den erfindungsgemäßen Produkten, bezogen auf die gesamte treibmittelfreie Wasser-in-ÖI-Emulsion, vorzugsweise in einer Gesamtmenge von 0,01 - 3, bevorzugt 0,1 - 1,5 und besonders bevorzugt 0,2 - 1,0 Gew.-% enthalten.
Die erfindungsgemäße Wasser-in-ÖI-Emulsion ist zusammen mit mindestens einem Treibmittel in einer Aerosol-Abgabevorrichtung enthalten; das erfindungsgemäße kosmetische Produkt besteht aus der Wasser-in-ÖI-Emulsion wie vorstehend dargestellt, mindestens einem Treibmittel und einer Aerosol-Abgabevorrichtung.
Erfindungsgemäß geeignete Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase.
Weitere erfindungsgemäß geeignete und bevorzugte Treibmittel sind ausgewählt aus mindestens einer Verbindung mit 3 bis 10 Kohlenstoffatomen gemäß Formel (I) 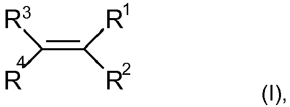 worin die Reste R¹, R², R³ und R⁴ unabhängig voneinander ein Wasserstoffatom, ein Bromatom, ein Fluoratom oder eine mit mindestens einem Fluoratom substituierte (C₁ bis C₆)-Alkylgruppe bedeuten, oder zwei der Reste R¹, R², R³ und R⁴ einen fünf- oder sechsgliedrigen Ring bilden, mit der Maßgabe, dass mindestens einer der Reste R¹, R², R³ oder R⁴ für ein Wasserstoffatom oder ein Fluoratom steht und mindestens einer der Reste R¹, R², R³ oder R⁴ für eine mit mindestens einem Fluoratom substituierte (C₁ bis C₆)-Alkylgruppe steht oder mindestens zwei der Reste R¹, R², R³ und R⁴ einen fünf- oder sechsgliedrigen Ring bilden.
Bevorzugte Treibmittel sind ausgewählt aus mindestens einer Verbindung mit 3 bis 10 Kohlenstoffatomen gemäß der vorstehenden Formel (I), in der R¹, R², R³ oder R⁴ unabhängig voneinander ein Wasserstoffatom, ein Fluoratom oder eine mit mindestens einem Fluoratom substituierte (C₁ bis C₆)-Alkylgruppe bedeuten, mit der Maßgabe, dass mindestens einer der Reste R¹, R², R³ oder R⁴ für ein Wasserstoffatom oder ein Fluoratom steht, und mindestens einer der Reste R¹, R², R³ oder R⁴ für eine mit mindestens einem Fluoratom substituierte (C₁ bis C₆)-Alkylgruppe steht.
Besonders bevorzugte Treibmittel sind ausgewählt aus Verbindungen der Formel *E*-R¹CH=CHR^{z} oder der Formel *Z*-R¹CH=CHR², worin R¹ und R² unabhängig voneinander eine perfluorierte C₁- bis C₆-Alkylgruppe darstellen.
Weitere besonders bevorzugte Treibmittel sind ausgewählt aus CF₃CF=CHF, CF₃CH=CF₂, CHF₂CF=CF₂, CHF₂CH=CHF, CF₃CF=CH₂, CF₃CH=CHF, CH₂FCF=CF₂, CHF₂CH=CF₂, CHF₂CF=CHF, CHF₂CF=CH₂, CF₃CH=CH₂, CH₃CF=CF₂, CH₂FCH=CF₂, CH₂FCF=CHF, CHF₂CH=CHF, CF₃CF=CFCF₃, CF₃CF₂CF=CF₂, CF₃CF=CHCF₃, CF₃CF₂CF=CH₂, CF₃CH=CHF₃, CF₃CF₂CH=CH₂, CF₂=CHCF₂CF₃, CF₂=CHCF₂CF₃, CF₂=CFCHFCF₃, CF₂=CFCF₂CHF₂, CHF₂CH=CHCF₃, (CF₃)₂C=CHCF₃, CF₃CF=CHCF₂CF₃, CF₃CH=CFCF₂CF₃, (CF₃)₂CFCH=CH₂, CF₃CF₂CF₂CH=CH₂, CF₃(CF₂)₃CF=CF₂, CF₃CF₂CF=CFCF₂CF₃, (CF₃)₂C=C(CF₃)₂, (CF₃)₂CFCF=CHCF₃, CF₂=CFCF₂CH₂F, CF₂=CFCHFCHF₂, CH₂=C(CF₃)₂, CH₂CF₂CF=CF₂, CH₂FCF=CFCHF₂, CH₂FCF₂CF=CF₂, CF₂=C(CF₃)(CH₃), CH₂=C(CHF₂)(CF₃), CH₂=CHCF₂CHF₂, CF₂=C(CHF₂)(CH₃), CHF=C(CF₃)(CH₃), CH₂=C(CHF₂)₂, CF₃CF=CFCH₃, CH₃CF=CHCF₃, CF₂=CF(CF₂)₂CF₃, CHF=CF(CF₂)₂CF₃, CF₂=CH(CF₂)₂CF₃, CF₂=CF(CF₂)₂CHF₂, CHF₂CF=CFCF₂CF₃, CF₃CF=CFCF₂CHF₂, CF₃CF=CFCHFCF₃, CHF=CFCF(CF₃)₂, CF₂=CFCH(CF₃)₂, CF₃CH=C(CF₃)₂, CF₂=CHCF(CF₃)₂, CH₂=CF(CF₂)₂CF₃, CHF=CF(CF₂)₂CHF₂, CH₂=C(CF₃)C₂F₅, CF₂=CHCH(CF₃)₂, CHF=CHCF(CF₃)₂, CF₂=C(CF₃)CH₂CF₃, CH₂=CF(CF₂)₂CHF₂, CF₂=CHCF₂CH₂CF₃, CF₃CF=C(CF₃)CH₃, CH₂=CFCH(CF₃)₂, CHF=CHCH(CF₃)₂, CH₂FCH=C(CF₃)₂, CH₃CF=C(CF₃)₂, CH₂=CHCF₂CHFCF₃, CH₂=C(CF₃)CH₂CF₃, (CF₃)₂C=CHC₂F₅, CH₂=CHC(CF₃)₃, (CF₃)₂C=C(CH₃)CF₃, CH₂=CFCF₂CH(CF₃)₂, CF₃CF=C(CH₃)C₂F₅, CF₃CH=CHCH(CF₃)₂, CH₂=CH(CF₂)₃CHF₂, (CF₃)₂C=CHCF₂CH₃, CH₂=C(CF₃)CH₂C₂F₅, CH₂=CHCH₂CF₂CF₂CF₃, C₂F₅CF=CFC₂H₅, CH₂=CHCH₂CF(CF₃)₂, CF₃CF=CHCH(CF₃)(CH₃), (CF₃)₂C=CFC₂H₅, cyclo-CF₂CF₂CF₂CH=CH-, cyclo-CF₂CF₂CH=CH-, CF₃CF₂CF₂C(CH₃)=CH₂, CF₃CF₂CF₂CH=CHCH₃, cyclo-CF₂CF₂CF=CF-, cyclo-CF₂CF=CFCF₂CF₂-, cyclo-CF₂CF=CFCF₂CF₂CF₂-, CF₃CF₂CF₂CF₂CH=CH₂, CF₃CH=CHC₂F₅, C₂F₅CH=CHC₂F₅, CF₃CH=CHCF₂CF₂CF₃, CF₃CF=CFC₂F₅, CF₃CF=CFCF₂CF₂CF₂CF₃, C₂F₅CF=CFCF₂CF₂CF₃, CF₃CH=CFCF₂CF₂CF₂CF₃, CF₃CF=CHCF₂CF₂CF₂CF₃, C₂F₅CH=CFCH₂CH₂CH₃, C₂F₅CF=CHCF₂CF₂CF₃, CF₃CF₂CF₂CF=CHCH₃, C₂F₅CF=CHCH₃, (CF₃)₂C=CHCH₃, CF₃C(CH₃)=CHCF₃, CHF=CFC₂F₅, CHF₂CF=CFCF₃, (CF₃)₂C=CHF, CH₂FCF=CFCF₃, CHF=CHC₂F₅, CHF₂CH=CFCF₃, CHF=CFCHFCF₃, CF₃CH=CFCHF₂, CHF=CFCF₂CHF₂, CHF₂CF=CFCHF₂, CH₂CF=CFCF₃, CH₂FCH=CFCF₃, CH₂=CFCHFCF₃, CH₂=CFCF₂CHF₂, CF₃CH=CFCH₂F, CHF=CFCH₂CF₃, CHF=CHCHFCF₃, CHF=CHCF₂CHF₂, CHF₂CF=CHCHF₂, CHF=CFCHFCHF₂, CF₃CF=CHCH₃, CF₂=CHCF₂Br, CHF=CBrCHF₂, CHBr=CHCF₃, CF₃CBr=CFCF₃, CH₂=CBrC₂F₅, CHBr=CHC₂F₅, CH₂=CH(CF₂)₂Br, CH₂=CHCBrFCF₃, CH₃CBr=CHCF₃, CF₃CBr=CHCH₃, (CF₃)₂C=CHBr, CF₃CF=CBrC₂F₅, E-CHF₂CBr=CFC₂F₅, Z-CHF₂CBr=CFC₂F₅, CF₂=CBrCHFC₂F₅, (CF₃)₂CFCBr=CH₂, CHBr=CF(CF₂)₂CHF₂, CH₂=CBrCF₂CF₂CF₃, CF₂=C(CH₂Br)CF₃, CH₂=C(CBrF₂)CF₃, (CF₃)₂CHCH=CHBr, (CF₃)₂C=CHCH₂Br, CH₂=CHCF(CF₃)CBrF₂, CF₂=CHCF₂CH₂CBrF₂, CFBr=CHCF₃, CFBr=CFCF₃ und CH₂=CBrCF₂CF₂CF₂CF₃, jeweils in der *E*-Form oder der *Z*-Form, sowie Mischungen der vorgenannten Komponenten.
Besonders bevorzugte erfindungsgemäße kosmetische Produkte enthalten als Treibmittel der Formel (I) das *E*-CF₃CH=CHF (*E*-1,3,3,3-Tetrafluorprop-1-en).
Erfindungsgemäß besonders bevorzugte Produkte sind dadurch gekennzeichnet, daß das/die Treibmittel in einer Menge von 10 - 90 Gew.%, bevorzugt 40 - 90 Gew.% und besonders bevorzugt 50 - 80 Gew.-%, jeweils bezogen auf das Gesamtgewicht der Zubereitung, bestehend aus der W/O-Emulsion und dem Treibmittel, enthalten ist/sind.
Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der Wasser-in-Öl-Emulsion.
Die erfindungsgemäßen Produkte weisen trotz der Wasserphase im Aerosolbehälter eine besonders hohe Korrosionsbeständigkeit auf, was einen großen Vorteil gegenüber dem Stand der Technik darstellt. Ferner weisen die erfindungsgemäß eingesetzten Wasser-in-ÖI-Emulsionen eine ausgezeichnete Hautverträglichkeit, Lagerstabilität und Wirksamkeit auf. Vorteilhaft ist insbesondere, dass die versprühten Produkte sich auf der Haut durch ein angenehmes, nicht-klebriges Hautgefühl mit einem lang anhaltenden Kühleffekt auszeichnen.
In einer bevorzugten Ausführungsform der Erfindung weist das Ventil einen mit einem Lack oder einem polymeren Kunststoff A beschichteten Ventilteller und ein ebensolches flexibles Element mit Rückstellcharakteristik auf, dass das Ventil nach Beenden der Betätigung in die Verschlussstellung (= Ruhelage des Ventils) zurückstellt. Entsprechende kosmetische Produkte, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff A beschichtet ist/sind, sind erfindungsgemäß bevorzugt.
In einer weiteren bevorzugten Ausführungsform der Erfindung weist das Ventil ein flexibles Element mit Rückstellcharakteristik und/oder einen Ventilteller aus mindestens einem Kunststoff B, bevorzugt einem elastomeren Kunststoff, auf. Auch hier sind erfindungsgemäße kosmetische Produkte, bei denen das Ventil ein flexibles Element mit Rückstellcharakteristik und/oder einen Ventilkegel aus mindestens einem Kunststoff B aufweist, bevorzugt, wobei bevorzugte Kunststoffe B elastomere Kunststoffe sind. Besonders bevorzugte elastomere Kunststoffe sind ausgewählt aus Buna, insbesondere Buna N, Buna 421, Buna 1602 und Buna KA 6712, Neopren, Butyl und Chlorbutyl.
In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element mit Rückstellcharakteristik einstückig mit dem Ventilkegel ausgebildet sein und biegsame Beine aufweisen. Diese Feder kann aus Metall, bevorzugt aus hoch vergütetem, korrosionsstabilen Edelstahl (z. B Sorte AISI 316) oder Kunststoff sein.
In einer besonders bevorzugten Ausführungsform der Erfindung sind Ventilkegel und flexibles Element mit Rückstellcharakteristik ausgebildet. Besonders bevorzugt ist dabei der Ventiltyp Ariane M, erhältlich von der Firma Seaquist Perfect, bei dem das flexible Element mit Rückstellcharakteristik in Form von vier elastischen Beinen einstückig mit dem Ventilkegel ausgebildet ist. Alle erfindungsgemäß verwendeten Ventile weisen vorzugsweise einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, müssen angesichts der Korrosivität der erfindungsgemäß verwendeten Wasser-in-ÖI-Emulsionen ebenfalls innen lackiert oder beschichtet sein. Ein erfindungsgemäß bevorzugter Innenschutzlack ist ein Epoxy-Phenollack, wie er u. a. unter den Bezeichnungen Hoba 7407 P und Hoba 7940 erhältlich ist.

Die nachfolgenden Beispiele sollen den Gegenstand der Erfindung verdeutlichen, ohne ihn hierauf zu beschränken.

Erfindungsgemäße Wasser-in-ÖI-Emulsionen (alle Mengenangaben in Gew.-%, bezogen auf das Gesamtgewicht der Wasser-in-ÖI-Emulsion ohne Treibmittel)

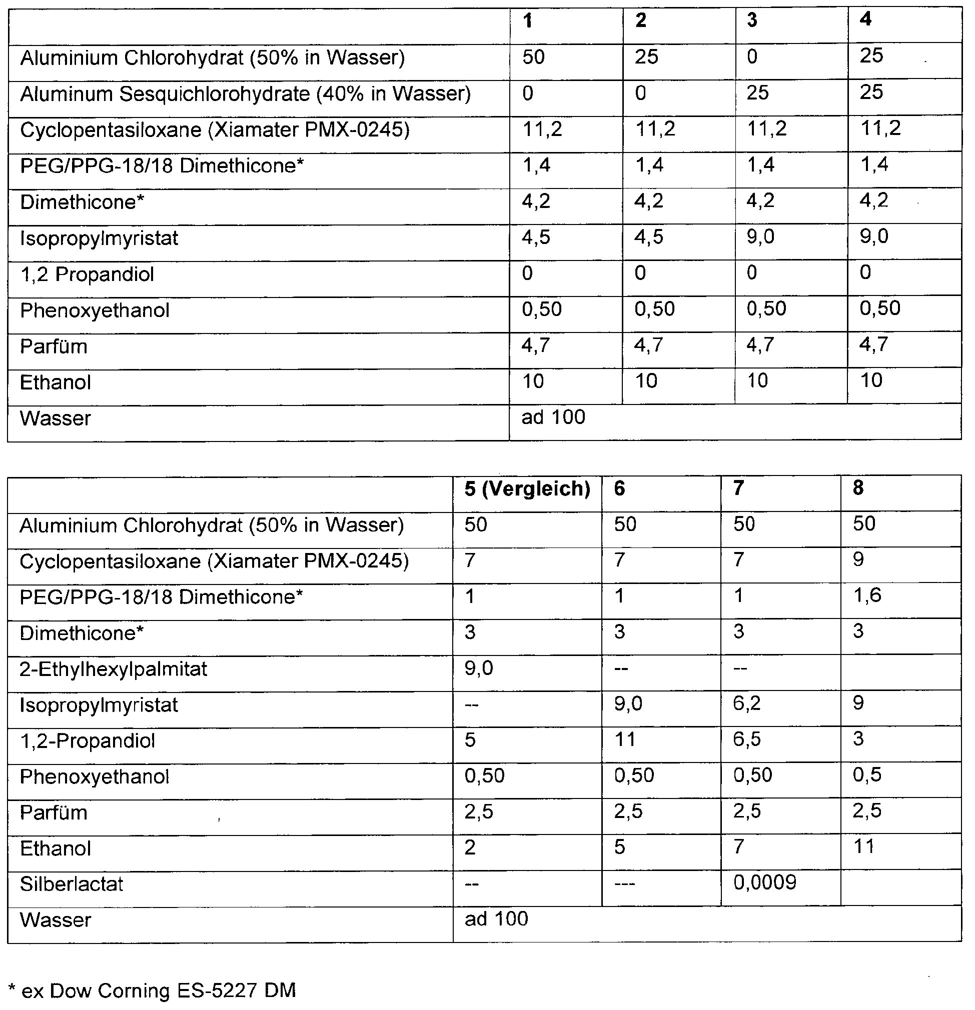

Die erfindungsgemäßen Wasser-in-Öl-Emulsionen Nr. 1 bis 4 und 6 bis 8 werden in eine innen mit Epoxy-Phenollack beschichtete Aluminiumspraydose in einem Gewichtsverhältnis von Treibmittel (Butan/Propan/ Isobutangemisch) zu Emulsion von 80:20 bzw. 85:15 bzw. 60:40 zu einem erfindungsgemäßen Produkt abgefüllt. Nach dem Aufsprühen auf die Achselhaut hinterlassen sie einen angenehm trockenen Kühleffekt.

## Patentansprüche

1. Kosmetisches Produkt, bestehend aus
- einer Wasser-in-Öl-Emulsion, enthaltend - bezogen auf das Gewicht der Emulsion -
a) 5 bis 35 Gew.-% mindestens eines schweißhemmenden Aluminiumsalzes,
b) 5 bis 14,25 Gew.-% Ethanol,
c) 0 bis 13 Gew.-% 1,2-Propandiol,
- mindestens einem Treibmittel,
- einer Aerosol-Abgabevorrichtung.

2. Produkt gemäß Anspruch 1, **dadurch gekennzeichnet, daß** die Emulsion 6 bis 32,5 Gew.-%, bevorzugt 7 bis 30 Gew.-%, besonders bevorzugt 8 bis 27,5 Gew.-%, außerordentlich bevorzugt 9 bis 26 Gew.-% und insbesondere 10 bis 25 Gew.-%, jeweils bezogen auf das Gesamtgewicht der kristallwasser- und ligandenfreien Aktivsubstanz (USP) in der treibmittelfreien Wasser-in-Öl-Emulsion, schweißhemmende(s) Aluminiumsalz(e) enthält.

3. Produkt nach einem der Ansprüche 1 oder 2, **dadurch gekennzeichnet, daß** die Emulsion 6 bis 14 Gew.-%, außerordentlich bevorzugt 7,5 bis 13,5 Gew.-% und insbesondere 10 bis 13 Gew.-% Ethanol enthält.

4. Produkt nach einem der Ansprüche 1 bis 3, **dadurch gekennzeichnet, daß** die Emulsion 0,5 bis 12 Gew.-%, bevorzugt 1 bis 11 Gew.-%, weiter bevorzugt 2 bis 10 Gew.-%, besonders bevorzugt 3 bis 9 Gew.-%, außerordentlich bevorzugt 4 bis 8 Gew.-% und insbesondere 5 bis 7 Gew.-% 1,2-Propandiol enthält.

5. Produkt nach einem der Ansprüche 1 bis 3, **dadurch gekennzeichnet, daß** die Emulsion weniger als 1 Gew.-%, bevorzugt weniger als 0,75 Gew.-%, weiter bevorzugt weniger als 0,5 Gew.-%, besonders bevorzugt weniger als 0,25 Gew.-%, außerordentlich bevorzugt weniger als 0,1 Gew.-% und insbesondere weniger als 0,01 Gew.-% 1,2-Propandiol enthält.

6. Produkt nach einem der Ansprüche 1 bis 5, **dadurch gekennzeichnet, daß** die Wasser-in-ÖI-Emulsion eine Ölphase von 1 - 60 Gew.-%, bezogen auf das Gesamtgewicht der Emulsion, enthält, wobei die Ölphase zu mindestens 90 Gew.-% aus bei 20 °C flüssigen Ölkomponenten besteht.

7. Produkt nach einem der Ansprüche 1 bis 6, **dadurch gekennzeichnet, daß** die Wasser-in-Öl-Emulsion 1 - 60 Gew.-%, vorzugsweise 2,5 bis 55 Gew.-%, weiter bevorzugt 5 bis 50 Gew.-%, besonders bevorzugt 7,5 bis 45 Gew.-% und insbesondere 10 bis 40 Gew.-% mindestens eines Öls aus der Gruppe Decamethylcyclopentasiloxan, Dodecamethylcyclohexasiloxan, Hexamethyldisiloxan (L₂), Octamethyltrisiloxan (L₃), Decamethyltetrasiloxan (L₄), beliebigen Zweier- und Dreiermischungen aus L₂, L₃ und/ oder L₄, weiterhin 2-Ethylhexylpalmitat, Hexyldecyllaurat, 2-Ethylhexylstearat, 2-Ethylhexyllaurat, Isopropylmyristat, Isopropylpalmitat, C₁₂-C₁₅-Alkylbenzoat, C₁₂-C₁₅-Alkyllactat, Di-C₁₂-C₁₃-Alkylmalat, PPG-14-Butylether, Isododecan, Isohexadecan, Isoeicosan, Polyisobuten und Polydecene sowie Mischungen der genannten Komponenten, enthält.

8. Produkt nach einem der Ansprüche 1 bis 7, **dadurch gekennzeichnet, daß** die Wasser-in-Öl-Emulsion mindestens einen Wasser-in-ÖI-Emulgator, ausgewählt aus der Gruppe von Poly-(C₂-C₃)alkylenglycol-modifizierten Siliconen mit den INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen) in einer Menge von 0,5 - 5 Gew.-%, bezogen auf das Gesamtgewicht der Emulsion, enthält.

9. Produkt nach einem der Ansprüche 1 bis 8, **dadurch gekennzeichnet, daß** das/die Treibmittel in einer Menge von 10 - 90 Gew.%, bevorzugt 40 - 90 Gew.% und besonders bevorzugt 50 - 80 Gew.-%, jeweils bezogen auf das Gesamtgewicht der Zubereitung, bestehend aus der W/O-Emulsion und dem Treibmittel, enthalten ist/sind.

## Claims

1. A cosmetic product, consisting of
- a water-in-oil emulsion containing, based on the weight of the emulsion,
a) 5 to 35 wt.% of at least one antiperspirant aluminum salt,
b) 5 to 14.25 wt.% ethanol,
c) 0 to 13 wt.% 1,2-propanediol,
- at least one propellant,
- an aerosol-dispensing device.

2. The product according to claim 1, **characterized in that** the emulsion contains 6 to 32.5 wt.%, preferably 7 to 30 wt.%, particularly preferably 8 to 27.5 wt.%, extremely preferably 9 to 26 wt.% and in particular 10 to 25 wt.%, antiperspirant aluminum salt(s), in each case based on the total weight of the active substance (USP), which is free of water of crystallization and ligands, in the propellant-free water-in-oil emulsion.

3. The product according to one of claims 1 or 2, **characterized in that** the emulsion contains 6 to 14 wt.%, extremely preferably 7.5 to 13.5 wt.% and in particular 10 to 13 wt.%, ethanol.

4. The product according to one of claims 1 to 3, **characterized in that** the emulsion contains 0.5 to 12 wt.%, preferably 1 to 11 wt.%, more preferably 2 to 10 wt.%, particularly preferably 3 to 9 wt.%, extremely preferably 4 to 8 wt.% and in particular 5 to 7 wt.%, 1,2-propanediol.

5. The product according to one of claims 1 to 3, **characterized in that** the emulsion contains less than 1 wt.%, preferably less than 0.75 wt.%, more preferably less than 0.5 wt.%, particularly preferably less than 0.25 wt.%, extremely preferably less than 0.1 wt.% and in particular less than 0.01 wt.%, 1,2-propanediol.

6. The product according to one of claims 1 to 5, **characterized in that** the water-in-oil emulsion contains an oil phase of 1 to 60 wt.%, based on the total weight of the emulsion, at least 90 wt.% of the oil phase consisting of oil components that are liquid at 20 °C.

7. The product according to one of claims 1 to 6, **characterized in that** the water-in-oil emulsion contains 1 to 60 wt.%, preferably 2.5 to 55 wt.%, more preferably 5 to 50 wt.%, particularly preferably 7.5 to 45 wt.% and in particular 10 to 40 wt.%, of at least one oil from the group decamethylcyclopentasiloxane, dodecamethylcyclohexasiloxane, hexamethyldisiloxane (L₂), octamethyltrisiloxane (L₃), decamethyltetrasiloxane (L₄), any binary or ternary mixtures of L₂, L₃ and/or L₄, in addition 2-ethylhexyl palmitate, hexyldecyl laurate, 2-ethylhexyl stearate, 2-ethylhexyl laurate, isopropyl myristate, isopropyl palmitate, C₁₂-C₁₅ alkyl benzoate, C₁₂-C₁₅ alkyl lactate, di-C₁₂-C₁₃ alkyl malate, PPG-14 butyl ether, isododecane, isohexadecane, isoeicosane, polyisobutene and polydecene and mixtures of the mentioned components.

8. The product according to one of claims 1 to 7, **characterized in that** the water-in-oil emulsion contains at least one water-in-oil emulsifier, selected from the group of poly(C₂-C₃)alkylene glycol-modified silicones having the INCI names PEG-x Dimethicone (where x = 2 to 20, preferably 3 to 17, particularly preferably 11 to 12), Bis-PEG-y Dimethicone (where y = 3 to 25, preferably 4 to 20), PEG/PPG a/b Dimethicone (where a and b represent, independently of one another, numbers from 2 to 30, preferably 3 to 30 and particularly preferably 12 to 20, in particular 14 to 18), Bis-PEG/PPG-c/d Dimethicone (where c and d represent, independently of one another, numbers from 10 to 25, preferably 14 to 20 and particularly preferably 14 to 16) and Bis-PEG/PPG-e/f PEG/PPG g/h Dimethicone (where e, f, g and h represent, independently of one another, numbers from 10 to 20, preferably 14 to 18 and particularly preferably 16) in an amount of from 0.5 to 5 wt.%, based on the total weight of the emulsion.

9. The product according to one of claims 1 to 8, **characterized in that** the propellant(s) is/are contained in an amount of from 10 to 90 wt.%, preferably 40 to 90 wt.% and particularly preferably 50 to 80 wt.%, in each case based on the total weight of the preparation, consisting of the W/O emulsion and the propellant.

## Revendications

1. Produit cosmétique constitué
- d'une émulsion eau-dans-huile contenant, par rapport au poids de l'émulsion,
a) de 5 à 35 % en poids d'au moins un sel d'aluminium anti-transpirant,
b) de 5 à 14,25 % en poids d'éthanol,
c) de 0 à 13 % en poids de 1,2-propanediol,
- d'au moins un agent propulseur,
- d'un dispositif de distribution sous forme d'aérosol.

2. Produit selon la revendication 1, **caractérisé en ce que** l'émulsion contient de 6 à 32,5 % en poids, de préférence de 7 à 30 % en poids, de façon particulièrement préférée de 8 à 27,5 % en poids, de façon extraordinairement préférée de 9 à 26 % en poids et en particulier de 10 à 25 % en poids de sel(s) d'aluminium anti-transpirant(s), dans chaque cas par rapport au poids total de la substance active exempte d'eau de cristallisation et de ligand (USP) dans l'émulsion eau-dans-huile exempte d'agent propulseur.

3. Produit selon l'une quelconque des revendications 1 ou 2, **caractérisé en ce que** l'émulsion contient de 6 à 14 % en poids, de façon extraordinairement préférée de 7,5 à 13,5 % en poids et en particulier de 10 à 13 % en poids d'éthanol.

4. Produit selon l'une quelconque des revendications 1 à 3, **caractérisé en ce que** l'émulsion contient de 0,5 à 12% en poids, de préférence de 1 à 11 % en poids, de façon plus préférée de 2 à 10 % en poids, de façon particulièrement préférée de 3 à 9 % en poids, de façon extraordinairement préférée de 4 à 8 % en poids et en particulier de 5 à 7% en poids de 1,2-propanediol.

5. Produit selon l'une quelconque des revendications 1 à 3, **caractérisé en ce que** l'émulsion contient moins de 1 % en poids, de préférence moins de 0,75 % en poids, de façon plus préférée moins de 0,5 % en poids, de façon particulièrement préférée moins de 0,25 % en poids, de façon extraordinairement préférée moins de 0,1 % en poids et en particulier moins de 0,01 % en poids de 1,2-propanediol.

6. Produit selon l'une quelconque des revendications 1 à 5, **caractérisé en ce que** l'émulsion eau-dans-huile contient une phase huileuse représentant de 1 à 60 % en poids, par rapport au poids total de l'émulsion, la phase huileuse étant constituée à hauteur d'au moins 90 % en poids de constituants huileux liquides à 20 °C.

7. Produit selon l'une quelconque des revendications 1 à 6, **caractérisé en ce que** l'émulsion eau-dans-huile contient de 1 à 60 % en poids, de préférence de 2,5 à 55 % en poids, de façon plus préférée de 5 à 50 % en poids, de façon particulièrement préférée de 7,5 à 45% en poids et en particulier de 10 à 40 % en poids d'au moins une huile du groupe constitué par le décaméthylcyclopentasiloxane, le dodécaméthylcyclohexasiloxane, l'hexaméthyldisiloxane (L₂), l'octaméthyltrisiloxane (L₃), le décaméthyltétrasiloxane (L₄), de mélanges de deux ou trois de L₂, L₃ et/ou L₄, ainsi que le palmityate de 2-éthylhexyle, le laurate d'hexyldécyle, le stéarate de 2-éthylhexyle, le laurate de 2-éthylhexyle, le myristate d'isopropyle, le palmitate d'isopropyle, le benzoate d'alkyle en C₁₂ à C₁₅, le lactate d'alkyle en C₁₂ à C₁₅, le malate de dialkyle en C₁₂ à C₁₃, le PPG14 butyléther, l'isododécane, l'isohexadécane, l'isoicosane, le polyisobutène, les polydécènes, et les mélanges de ces constituants.

8. Produit selon l'une quelconque des revendications 1 à 7, **caractérisé en ce que** l'émulsion eau-dans-huile contient au moins un émulsifiant eau-dans-huile choisi dans le groupe constitué par les silicones modifiées au glycol de polyalkylène en C₂ à C₃ portant les désignations INCI PEG-x Dimethicone (où x est un nombre situé dans la plage allant de 2 à 20, de préférence de 3 à 17, et de façon plus préférée de 11 à 12), Bis-PEG-y Dimethicone (où y est un nombre situé dans la plage allant de 3 à 25, et de préférence de 4 à 20), PEG/PPG-a/b Dimethicone (où a et b représentent indépendamment l'un de l'autre des nombres situés dans la plage allant de 2 à 30, de préférence de 3 à 30 et de façon plus préférée de 12 à 20, en particulier de 14 à 18), Bis-PEG/PPG-c/d Dimethicone (où c et d représentent indépendamment l'un de l'autre des nombres situés dans la plage allant de 10 à 25, de préférence de 14 à 20 et de façon particulièrement préférée de 14 à 16), et Bis-PEG/PPG-e/f PEG/PPG-g/h Dimethicone (où e, f, g et h représentent indépendamment les uns des autres des nombres situés dans la plage allant de 10 à 20, de préférence de 14 à 18 et de façon particulièrement préférée 16) en une quantité située dans la plage allant de 0,5 à 5 % en poids, par rapport au poids total de l'émulsion.

9. Produit selon l'une quelconque des revendications 1 à 8, **caractérisé en ce que** le ou les agents propulseur(s) est/sont présent(s) en une quantité située dans la plage allant de 10 à 90 % en poids, de préférence de 40 à 90 % en poids et de façon particulièrement préférée de 50 à 80 % en poids, dans chaque cas par rapport au poids total de la préparation, constituée de l'émulsion eau-dans-huile et de l'agent propulseur.
